# EUROPEAN PATENT SPECIFICATION

(11) **EP 3 490 626 B1**
(45) Date of publication and mention of the grant of the patent: **13.05.2020**
(21) Application number: 17751324.9
(22) Date of filing: 28.07.2017
(51) Int. Cl.: A61L 27/60, C12N 5/071, G01N 33/50

(54) **SKIN EQUIVALENT WITH DISTINCT JUXTAPOSED DERMAL COMPARTMENTS**
HAUTÄQUIVALENT MIT VERSCHIEDENEN NEBENEINANDER LIEGENDEN DERMALEN KOMPARTIMENTEN
ÉQUIVALENT DE PEAU À COMPARTIMENTS DERMIQUES JUXTAPOSÉS DISTINCTS

(30) Priority: 29.07.2016 FR 1657418
(43) Date of publication of application: 05.06.2019
(73) Proprietor: L'Oreal, 75008 Paris (FR)
(72) Inventor: ASSELINEAU, Daniel, 93601 Aulnay-sous-Bois (FR); PAGEON, Hervé, 93601 Aulnay-sous-Bois (FR); RICOIS, Sylvie, 93601 Aulnay-sous-Bois (FR)
(74) Representative: Lavoix
(86) International application number: PCT/EP2017/069203
(87) International publication number: WO 2018/020016

(56) References cited:
- EP-A1- 1 046 402
- WO-A1-97/41208
- FR-A1- 2 930 644
- BERNERD F ET AL: "An organotypic model of skin to study photodamage and photoprotection in vitro", JOURNAL OF THE AMERICAN ACADEMY OF DERMATOLOGY, MOSBY, INC, US, vol. 58, no. 5, 1 May 2008 (2008-05-01), pages S155-S159, XP022617431, ISSN: 0190-9622, DOI: 10.1016/J.JAAD.2007.08.050 [retrieved on 2008-04-12]

## Description

This invention relates to new skin equivalents with surface heterogeneity.

For many years, it has been attempted to develop reconstructed skin models resembling human or animal skin as closely as possible, in order to avoid experimental assays on animals.

It is thus possible to reproduce a dermis equivalent by mixing collagen and fibroblasts under conditions leading to the formation of dermal tissue. This step can then be followed by skin reconstruction, by allowing keratinocytes to grow on this substrate in order to generate an epidermis. EP1046402 discloses reconstructed skin models for aged-skin studies, FR2930644 for skin pigmentation evaluation.

These conventional reconstructed skins, however, are homogeneous and not yet sufficiently similar to human skin, which is complex and heterogeneous.

There is thus a strong need for new reconstructed skins capable of reproducing the heterogeneous nature of skin.

This need has been met by the present invention.

Indeed, the present inventors have developed a dermis equivalent comprising distinct longitudinally juxtaposed dermal compartments, thus enabling addressing the issue of skin heterogeneity not in depth direction, but rather in length direction.

They indeed demonstrated that by focusing on distinct dermal compartments, it is possible to obtain a skin equivalent displaying surface heterogeneity thanks to the numerous interactions existing between dermis and epidermis, the quality of the dermis influencing that of the epidermis, and consequently the appearance of the epidermis and of the surface skin.

They thus demonstrated that a dermis subdivided into longitudinally distinct compartments enabled addressing local epidermal differences in terms of epidermal differentiation and keratinization, giving rise to surface effects, thus allowing the study of skin heterogeneity-related issues.

The inventors demonstrated that it is possible to prepare dermises displaying longitudinally distinct dermal compartments, distinguished by the dermal sub-populations present (papillary and reticular fibroblasts, or any other fibroblast population), or by the characteristics of the collagen used (glycated or not, or having undergone any other oxidative modification).

The present invention therefore relates to a dermis equivalent longitudinally comprising at least two distinct, juxtaposed dermal compartments with different compositions.

The present invention also relates to a skin equivalent displaying surface heterogeneity comprising a dermis equivalent according to the invention.

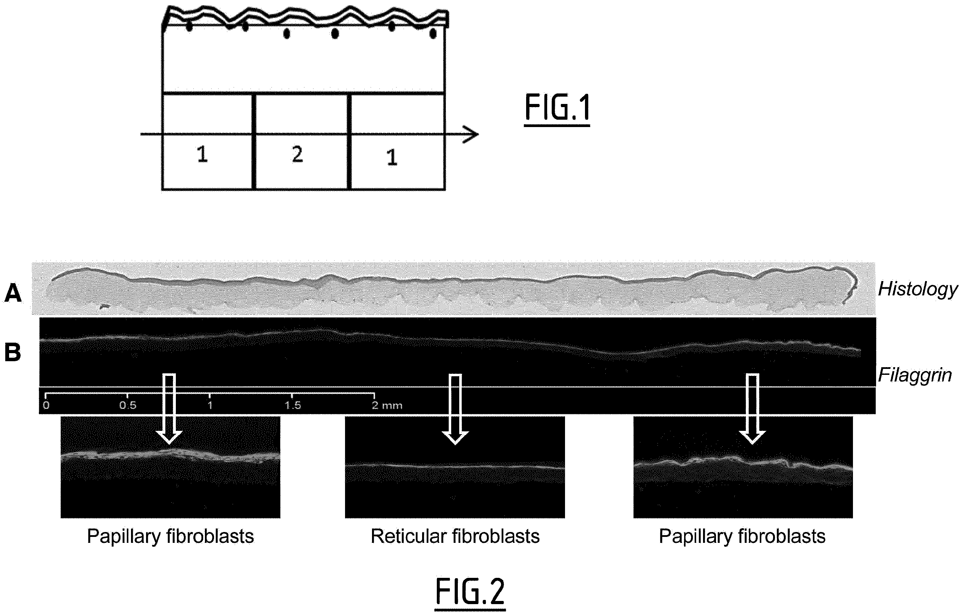

An example of skin equivalent according to the invention, the dermis equivalent of which comprises longitudinally three distinct juxtaposed dermal compartments with different compositions, is presented in Figure 1.

Another object of the invention pertains to a process for the preparation of a dermis equivalent according to the invention, comprising a step consisting in preparing at least two distinct, longitudinally juxtaposed lattices of different compositions, one of the at least two lattices being prepared inside a mold, while the other is prepared on the periphery of the mold.

Another object of the invention relates to a process for the preparation of a skin equivalent according to the invention, comprising a step of preparing a dermis equivalent by the process for the preparation of a dermis equivalent according to the invention.

The present invention also relates to the use of a dermis equivalent according to the invention for the preparation of a skin equivalent displaying surface heterogeneity.

Another object of the invention relates to the use, preferably *in vitro,* of a dermis equivalent according to the invention, or of a skin equivalent according to the invention to study skin heterogeneities or skin heterogeneity-related disorders, such as skin aging and/or inflammation and/or pigmentation.

Another object of the invention relates to a process of screening for a compound presenting an activity following topical application onto the skin, in particular onto skin displaying heterogeneities, particularly in the field of anti-aging, such as for the treatment of wrinkles and lines and/or in the field of inflammation and/or in the field of pigmentation, such as for example for the treatment of pigmentation spots, said screening process comprising the application of a candidate compound onto the dermis equivalent according to the invention, or onto the skin equivalent according to the invention.

### Detailed description of the invention

### Dermis equivalent and process of preparation

By "dermal compartment" is meant herein an area of dermis equivalent comprising fibroblasts and collagen.

Thus, the dermis equivalent according to the invention comprises longitudinally at least two distinct juxtaposed dermal compartments, comprising in particular fibroblasts and collagen, but of different compositions.

The dermis equivalent according to the invention comprises, longitudinally, at least two, preferably at least 3 distinct juxtaposed dermal compartments, of different compositions.

By "juxtaposed dermal compartments" is meant herein that the dermal compartments are in contact with each other via one of their lateral sides.

By "distinct compartments of different compositions" is meant herein that the dermal compartments of the dermis equivalent can be distinguished one from the other by their composition, in particular by the nature of the cells and in particular of the fibroblasts, comprised in the compartment and/or by the nature of the collagen comprised in the compartment and/or by the treatment applied to one of the compartment's constituents.

The collagen present in the dermal compartments of the dermis equivalent according to the invention can be of any type and of any origin. Preferably, the collagen is selected among the type I, III or V fibrillar collagens. Preferably, the collagen is of type I. Preferably, the collagen is of animal origin, in particular of bovine origin. Particularly preferably, the collagen is bovine type I collagen. Alternatively, the collagen can be a mixture of different types of collagen, in any proportions and/or of various origins.

In a particular embodiment, one of the dermal compartments comprises collagen that underwent oxidative modification and another of the dermal compartments comprises collagen that did not undergo oxidative modification, or that underwent another oxidative modification, different from that of the first compartment.

Some examples of oxidative modification include glycation, carbonylation and carbamylation.

In a particular embodiment, one of the dermal compartments comprises glycated collagen and another of the dermal compartments comprises non-glycated collagen.

By "glycated collagen" is meant herein collagen that has undergone glycation, i.e. collagen that reacted according to the Maillard reaction with an ose (in particular glucose or ribose) to form a Schiff base that, after a so-called Amadori molecular rearrangement, can lead, via a succession of reactions, to intramolecular bridge formation such as pentosidine bridges for example.

Various methods for monitoring glycation products formation are well-known to persons skilled in the art, such as the methods described in Cefalu et al. (1995) J. Gerontol A Biol Sci Med Sci 50A:B337-B341, in Sell et al. (1991) Diabetes Metab. Rev. 7:239-251, in Miyata et al. (1996) J. Am. Soc. Nephrol. 7:1198-1206 or in Ahmed et al. (1991) Anal. Biochem. 192:109-111. It is thus possible to measure the level of glycating compound bound to the collagen and/or the level of glycating compound remaining after the reaction. As previously described, collagen glycation leads to the formation of glycation products or advanced glycosylation end products (AGEs), such as pyrraline, carboxymethyl-lysine, pentosidine, N^{ε}(2-carboxyethyl)-lysine (CEL), glyoxal-lysine dimer (GOLD), methylglyoxal-lysine dimer (MOLD), 3DG-ARG imidazolone or glucosepane for example. Some of these glycation products have the particularity of emitting measurable fluorescence upon excitation. For example, pentosidine, when excited at a wavelength (λₑₓ) of 328 nm, emits fluorescence at a wavelength (λₑₘ) of 378 nm. Similarly, AGEs, when excited at a wavelength (λₑₓ) of 370 nm, emit fluorescence at a wavelength (λₑₘ) of 440 nm. Typically, the ratio of fluorescence emitted by a given glycation product in a dermal compartment comprising at least glycated collagen and fibroblasts to the fluorescence emitted by the same glycation product in a dermal compartment comprising at least non-glycated collagen and fibroblasts, measured under the same experimental conditions, allows the glycation level of the dermal compartment comprising glycated collagen to be characterized. Preferably, in the context of the invention, the glycation level of the dermal compartment comprising glycated collagen is determined by measuring pentosidine and/or AGE fluorescence. The glycated collagen can be obtained by any technique well-known to persons skilled in the art, in particular by pre-incubating collagen with a sugar, in particular ribose or glucose, prior to use for preparing the dermal compartment.

The fibroblasts present in the dermal compartments of the dermis equivalent according to the invention can be of any origin, though they are preferably fibroblasts of human origin. They can be prepared by any method well-known to persons skilled in the art, for example by mechanical and/or enzymatic dissociation of dermal extracellular matrix macromolecules, or by growth of fibroblasts from explants.

The fibroblasts present in the dermal compartments of the dermis equivalent according to the invention can in particular be papillary fibroblasts and/or reticular fibroblasts.

By "papillary fibroblasts" is meant herein fibroblasts from the papillary dermis.

By "reticular fibroblasts" is meant herein fibroblasts from the reticular dermis.

In a particular embodiment of the invention, one of the dermal compartments comprises papillary fibroblasts and another of the dermal compartments comprises reticular fibroblasts.

The dermal compartments of the dermis equivalent according to the invention can also comprise any other component that can be constitutively present in skin, such as endothelial cells, macrophages, monocytes, macrophage precursors, dendritic cell precursors or nervous cells.

The dermis equivalent according to the invention comprises, lengthwise, at least two distinct juxtaposed dermal compartments of different compositions. Moreover, it may display vertically differences in composition. In particular, it may include various layers of dermal compartments of different compositions.

The present invention also relates to a process for the preparation of a dermis equivalent as defined above, comprising a step consisting in preparing at least two distinct, longitudinally juxtaposed lattices of different compositions, one of the at least two lattices being prepared inside a mold, while the other is prepared on the periphery of the mold.

The at least two lattices comprise collagen and fibroblasts as defined above, but with display different compositions.

Preferably, the preparation step for the at least two lattices comprises the preparation of at least two distinct solutions of different compositions, one being for the interior of the mold, the other being for the periphery of the mold.

The at least two solutions used to prepare the lattices comprise collagen and a cell suspension of fibroblasts, and display different compositions.

In one embodiment, one of the at least two solutions comprise a cell suspension of papillary fibroblasts and the other of the at least two solutions comprises a cell suspension of reticular fibroblasts, as defined above.

In another embodiment, one of the at least two solutions comprises glycated collagen, while the other of the at least two solutions comprises non-glycated collagen, as defined above.

Preferably, the at least two solutions further comprise MEM 1.76X medium, FCS, NaOH and MEM 25 mM Hepes 10% FCS medium.

As previously mentioned, the collagen used can be any type of collagen, from any origin, either alone or in mixture.

The solution can comprise collagen at a concentration of between 2 mg/ml and 6 mg/ml, preferably between 3 mg/ml and 5 mg/ml.

When glycated collagen is used, glycation can be performed by any technique well-known to persons skilled in the art, such as for example that described in French patent application FR2792650.

Preferably, the solution comprises fibroblasts at a concentration of 1×10⁵ to 5×10⁶ cells/ml, preferably at a concentration of 2×10⁵ to 2×10⁶ cells/ml.

In the context of the invention, the at least two lattices are prepared on both sides of a mold: one of the at least two lattices is prepared inside a mold and the other is prepared at the periphery of, or outside, the mold.

Each of the lattices prepared on either side of the mold can be prepared by any technique well-known to persons skilled in the art.

In particular, each solution as defined above, comprising collagen and a cell suspension of fibroblasts, can be deposited onto a substrate comprising the mold.

Preferably, the solutions are incubated in such a manner as to enable collagen gelling, for example for 10 to 30 min.

Preferably the mold is removed once lattice gelling is obtained, and the lattices are preferably maintained in incubation to allow them to contract. Preferably, the lattices are thus maintained in incubation during 1 to 7 days, even more preferably for 3 days.

The mold used in the context of the invention can be of any shape and of any material suitable for cell culture. The mold used in the context of the invention can thus be rectangular or circular. The mold used in the context of the invention is preferably made from Teflon.

Insofar as the dermal content influences differentiation of the epidermal compartment, the dermis equivalent comprising longitudinally at least two distinct juxtaposed dermal compartments according to the invention, can serve as a substrate for the formation of a skin equivalent presenting surface heterogeneity induced by the different underlying dermal compartments.

Accordingly, the present invention also relates to the use of a dermis equivalent according to the invention for the preparation of a skin equivalent displaying surface heterogeneity.

### Skin equivalent

The present invention also relates to a skin equivalent displaying surface heterogeneity, comprising a dermis equivalent as defined in the section *"Dermis equivalent and preparation process"* above.

By "surface heterogeneity" is meant herein that the surface of the skin equivalent displays properties or characteristics that vary along its length, e.g.: differences in pigmentation, in differentiation of the surface epidermal zone, in surface suppleness, etc.

The skin equivalent according to the invention comprises, on the dermis equivalent, an epidermis equivalent comprising at least keratinocytes.

The keratinocytes can be of any origin, but are preferably keratinocytes of human origin. They can be prepared by any method well-known to persons skilled in the art. Accordingly, the keratinocytes can be prepared by culturing dissociated epidermis from normal skin samples, or by culturing keratinocytes from the sheath of a hair follicle.

Preferably the keratinocytes are normal human skin keratinocytes.

Even more preferably, the keratinocytes are prepared from dissociated human epidermis from a normal skin sample according to the method described in Régnier et al., Frontier of Matrix Biology, Vol. 9, 4-35 (Karger, Basel, 1981).

The epidermis equivalent can comprise any other cell type, such as Langerhans cells and/or Langerhans cell precursors and/or melanocytes.

Advantageously, the epidermis equivalent further comprises melanocytes and/or Langerhans cells and/or Langerhans cell precursors.

The melanocytes can be isolated from any organ containing them, such as normal skin or hair follicle. Preferentially, the melanocytes are isolated from normal skin. Any method of preparation of melanocytes well-known to persons skilled in the art may be used, such as the method described in Olsson et al. (1994) Acta. Derm. Venereol. 74:226-268.

The Langerhans cells and/or Langerhans cell precursors can be as described in European patent application EP 789074.

Preferably, the epidermis equivalent is not longitudinally compartmented.

By "non longitudinally compartmented epidermis equivalent" is meant herein that the epidermis equivalent is prepared from a single composition seeded onto the at least two distinct juxtaposed dermal compartments. It should however be noted that, as the dermis and epidermis interact strongly, the differences in the distinct juxtaposed dermal compartments may induce differences longitudinally in the properties of the formed epidermis equivalent.

The present invention also relates to a process for preparing a skin equivalent as defined above, comprising a step of preparation of a dermis equivalent by the process of preparation of a dermis equivalent as defined in the section *"Dermis equivalent and preparation process".*

Preferably, the process of preparation of a skin equivalent comprises, after the step of preparation of the dermis equivalent, a step of reconstitution, on the dermis equivalent, of an epidermis equivalent, comprising at least keratinocytes.

This step of reconstitution of an epidermis equivalent can be performed by any technique well-known to persons skilled in the art, such as the techniques described in patent applications EP 285471, EP285474, EP789074, EP502172, EP418035, WO91/16010, EP197090, EP20753, FR2665175 and FR2689904, or that described in Asselineau et al. (1985) Exp. Cell. Res. 159:536-539, in Asselineau et al. (1987), Models in dermato., col III, Ed. Lowe&Mailbach, 1-7 or in Asselineau et al. (1984) Br J Dermatol. 111 Suppl 27:219-22.

This reconstitution step can be advantageously preceded by a collage step, in a culture dish, of the prepared dermis equivalent, for example using an adhesive solution comprising MEM 1.76X medium, FCS, NaOH 0.1N and MEM 25 mM Hepes 10% FCS.

Preferably, the reconstitution step is implemented by seeding keratinocytes onto the dermis equivalent, preferably into a seeding ring.

After seeding the keratinocytes onto the dermis equivalent, the culture can advantageously be held submerged in nutrient medium, which may for example be the medium described by Rheinwald and Green (1975) Cell 6:317-330, which enables keratinocyte proliferation.

Following an incubation period, preferably of 3 to 15 days, even more preferably of 7 to 9 days, the skin equivalent is preferably maintained at the air/liquid interface, for example by depositing it onto a metal mesh. This liquid is then preferably constituted of the same nutrient medium as previously.

Incubation is then continued, preferably until a skin equivalent displaying the characteristics of skin is obtained, i.e. a dermis equivalent covered by an epidermis equivalent displaying the four standard cell layers, namely the stratum basale, stratum suprabasale, stratum granulosum and stratum corneum. In this way, incubation is preferably continued for a duration of between 5 and 30 days, preferably between 7 and 10 days.

### Uses

The present invention also relates to the use of a dermis equivalent as defined in the section *"Dermis equivalent and preparation process"* above, or of a skin equivalent as defined in the section *"Skin equivalent and preparation process"* above, to study skin heterogeneities or skin heterogeneity-related disorders such as skin aging and/or inflammation and/or pigmentation.

In particular, the dermis equivalent and/or the skin equivalent according to the invention are useful for studying the onset of wrinkles or of pigmentation spots, or any other skin aging- and/or skin pigmentation-related phenomena, in particular associated with photo-aging, more specifically associated with the effect of ultraviolet radiation on the skin, such as actinic keratosis.

The present invention also relates to a process of screening for a compound presenting an activity following topical application onto the skin, in particular onto skin displaying heterogeneities, particularly in the field of anti-aging, such as for the treatment of wrinkles and lines and/or in the field of inflammation and/or in the field of pigmentation, such as for example for the treatment of pigmentation spots, said screening process comprising the application of a candidate compound onto the dermis equivalent according to the invention, or onto the skin equivalent according to the invention.

The present invention will be illustrated in more detail by the figures and example hereinbelow.

### Brief description of the figures

**Figure 1****:** Schematic representation of the skin equivalent according to the invention, comprising the dermis equivalent, longitudinally comprising at least two distinct juxtaposed dermal compartments of different compositions according to the invention.
**Figure 2****:** (A) Histology of the skin equivalent obtained in the example, comprising 3 distinct juxtaposed dermal compartments: one compartment comprising reticular fibroblasts surrounded by two compartments comprising papillary fibroblasts. (B) Labeling of the filaggrin present in the skin equivalent obtained in the example.

### Example

This example shows a reconstructed skin displaying alternately, at the dermal level, zones with papillary or reticular fibroblasts.

### Preparation of the dermis equivalent with three distinct compartments

Two human fibroblasts solutions, one comprising papillary fibroblasts, the other comprising reticular fibroblasts, are prepared in MEM 25 mM Hepes medium with 10% FCS at a rate of 2×10⁶ cells/ml.

From these fibroblast solutions, two distinct lattice preparation solutions are prepared, one (solution A) for the periphery of a 1.5 cm × 3.8 cm rectangular Teflon inclusion mold, and the other (solution B) for the interior of this mold.

Solution A comprises 3.22 ml of MEM 1.76X medium, 0.63 ml FXS, 0.35 ml NaOH 0.1 N, 0.2 ml of MEM 25 mM Hepes medium, 10% FCS and 0.5 ml of papillary fibroblasts solution.

Solution B comprises 3.22 ml MEM 1.76X medium, 0.63 ml FXS, 0.35 ml NaOH 0.1 N and 0.2 ml of MEM 25 mM Hepes medium, 10% FCS and 0.5 ml reticular fibroblasts solution.

2.1 ml of cold acid-soluble collagen I solution (5 mg/ml) is slowly added to solutions A and B.

The solutions are mixed with a pipette until homogenization.

The rectangular mold is placed at the center of a 4 cm × 4 cm square microscope dish covered with a solution comprising 3.22 ml of MEM 1.76X medium, 0.63 ml FXS, 0.35 ml NaOH 0.1 N and 0.2 ml of MEM 25 mM Hepes medium, 10% FCS. The assembly is incubated at 37°C.

1.7 ml of solution B is poured into the mold and 5.3 ml of solution A is poured at the mold's periphery. The assembly is then incubated for 15 to 20 min, to allow gelling to occur.

Once gelling is obtained, the mold is removed and the dish transferred to 37°C, 5% CO₂.

The preparation is then incubated at 37°C, 5% CO₂ for 3 days to allow the lattices to contract.

### Skin equivalent preparation

Seeding with keratinocytes to obtain the complete skin equivalent is performed after 3 days of lattice contraction.

An adhesive solution is prepared by mixing 0.46 ml of MEM 1.76X medium, 0.09 ml FCS, 0.05 ml NaOH 0.1N, 0.1 ml MEM Hepes 10% SVF medium and 0.3 ml dialyzed collagen.

A drop of this solution is deposited in the middle of a culture dish. The lattices comprising the 3 dermal compartments prepared above are taken and placed onto the drop of adhesive solution, then incubated in the oven at 37°C, 5% CO₂ for 20-30 min.

The keratinocytes are put in solution in MEM 10% FCS + 3F medium at a concentration of 1×10⁵ cells/ml.

A seeding ring is placed onto the bonded lattices and 0.5 ml of keratinocyte cell suspension is deposited into this ring. MEM 10% FCS+3F medium is added around the ring.

The assembly is then incubated at 37 °C, 5% CO₂ for 2 h.

The seeding ring is then removed and the dishes re-incubated at 37 °C, 5% CO₂ for 7 days, the culture medium being changed twice per week.

After 7 days of immersion culture, the skins are emerged: the adhesive around the skin to emerge is cut and the skin equivalent transferred to an emersion mesh in a dish comprising MEM 10% FCS + 3F medium.

The dishes are incubated at 37°C, 5% CO₂ for 7 days, the culture medium being changed twice per week.

### Analysis of the obtained skin equivalents

The obtained skin equivalent was studied by histology and labeled to detect filaggrin. As shown in **Figure 2****,** it is apparent in the histologies corresponding to the zones of dermal compartment with papillary fibroblasts and of dermal compartment with reticular fibroblasts, that the epidermal areas corresponding to these compartments can be distinguished by the quality of their differentiation, which is less pronounced for the reticular fibroblast compartment by comparison to the papillary fibroblast compartment.

This is confirmed, in the stratum granulosum, by the filaggrin labeling, which is stronger in the presence of a dermal part containing papillary fibroblasts, compared to a dermal part containing reticular fibroblasts.

## Claims

1. Dermis equivalent comprising longitudinally at least two distinct, juxtaposed dermal compartments of different compositions.

2. Dermis equivalent according to claim 1, wherein at least one of the at least two dermal compartments comprises collagen that has undergone oxidative modification and at least another of the at least two dermal compartments comprises collagen that has not undergone oxidative modification, or that has undergone another oxidative modification different from the first compartment.

3. Dermis equivalent according to claim 1 or 2, wherein at least one of the at least two dermal compartments comprises papillary fibroblasts and at least another of the at least two dermal compartments comprises reticular fibroblasts.

4. Skin equivalent displaying surface heterogeneity, comprising a dermis equivalent according to any one of claims 1 to 3.

5. Process for the preparation of a dermis equivalent according to any one of claims 1 to 3, comprising a step consisting in preparing at least two distinct, longitudinally juxtaposed lattices of different compositions, one of the at least two lattices being prepared inside a mold, while the other is prepared on the periphery of the mold.

6. Preparation process according to claim 5, wherein the step of preparation of the at least two lattices comprises the preparation of at least two distinct solutions of different compositions, one being for the inside the mold and the other being for the periphery of the mold.

7. Process of preparation of a skin equivalent according to claim 4, comprising a step of preparation of a dermis equivalent by the process according to claim 5 or 6.

8. Use of a dermis equivalent according to any one of claims 1 to 3 for the preparation of a skin equivalent displaying surface heterogeneity.

9. Use of a dermis equivalent according to any one of claims 1 to 3, or of a skin equivalent according to claim 4, to study skin heterogeneities such as skin aging and/or inflammation and/or pigmentation.

10. Process of screening for a compound displaying activity following topical application onto the skin, in particular onto skin displaying heterogeneities, particularly in the field of anti-aging, such as for the treatment of wrinkles and lines and/or in the field of inflammation and/or in the field of pigmentation, such as for example for the treatment of pigmentation spots, said screening process comprising the application of a candidate compound onto the dermis equivalent according to any one of claims 1 to 3, or onto the skin equivalent according to claim 4.

## Patentansprüche

1. Dermisäquivalent, in einer Längsrichtung mindestens zwei voneinander verschiedene, nebeneinander liegende dermale Bereiche mit unterschiedlichen Zusammensetzungen umfassend.

2. Dermisäquivalent nach Anspruch 1, wobei mindestens einer von den mindestens zwei dermalen Bereichen Kollagen umfasst, das einer oxidativen Modifikation unterzogen wurde, und mindestens ein anderer von den mindestens zwei dermalen Bereichen Kollagen umfasst, das keiner oxidativen Modifikation unterzogen wurde oder das einer anderen oxidativen Modifikation unterzogen wurde, die von der des ersten Bereichs verschieden ist.

3. Dermisäquivalent nach Anspruch 1 oder 2, wobei mindestens einer von den mindestens zwei dermalen Bereichen papilläre Fibroblasten umfasst und mindestens ein anderer von den mindestens zwei dermalen Bereichen retikuläre Fibroblasten umfasst.

4. Hautäquivalent, das eine Oberflächenheterogenität zeigt und das ein Dermisäquivalent nach einem der Ansprüche 1 bis 3 umfasst.

5. Verfahren zur Herstellung eines Dermisäquivalents nach einem der Ansprüche 1 bis 3, einen Schritt umfassend, der die Herstellung von mindestens zwei voneinander verschiedenen, in einer Längsrichtung nebeneinanderliegenden Gittern unterschiedlicher Zusammensetzungen beinhaltet, wobei eines von den mindestens zwei Gittern innerhalb einer Form hergestellt wird, während das andere am Außenrand der Form hergestellt wird.

6. Herstellungsverfahren nach Anspruch 5, wobei der Schritt des Herstellens der mindestens zwei Gitter das Herstellen von mindestens zwei voneinander verschiedenen Lösungen mit unterschiedlichen Zusammensetzungen umfasst, wobei eine für das Innere der Form und die andere für den Außenrand der Form bestimmt ist.

7. Verfahren zum Herstellen eines Dermisäquivalents nach Anspruch 4, einen Schritt des Herstellens eines Dermisäquivalents anhand des Verfahrens nach Anspruch 5 oder 6 umfassend.

8. Verwendung eines Dermisäquivalents nach einem der Ansprüche 1 bis 3 für die Herstellung eines Hautäquivalents, das eine Oberflächenheterogenität zeigt.

9. Verwendung eines Dermisäquivalents nach einem der Ansprüche 1 bis 3 oder eines Hautäquivalents nach Anspruch 4, um Hautheterogenitäten, wie etwa Hautalterung und/oder -entzündung und/oder - pigmentierung zu untersuchen.

10. Verfahren zum Erkennen einer Verbindung, die im Anschluss an eine topische Auftragung auf die Haut, insbesondere auf eine Haut, die Heterogenitäten zeigt, eine Wirkung zeigt, insbesondere auf dem Gebiet der Bekämpfung von Alterungszeichen, wie etwa zur Behandlung von Fältchen und Linien und/oder auf dem Gebiet der Entzündung und/oder auf dem Gebiet der Pigmentierung, wie beispielsweise für die Behandlung von Pigmentflecken, wobei das Erkennungsverfahren das Auftragen einer zu prüfenden Verbindung auf das Dermisäquivalent nach einem der Ansprüche 1 bis 3 oder auf das Hautäquivalent nach Anspruch 4 umfasst.

## Revendications

1. Equivalent de derme comprenant longitudinalement au moins deux compartiments dermiques juxtaposés distincts de compositions différentes.

2. Equivalent de derme selon la revendication 1, dans lequel au moins un des au moins deux compartiments dermiques comprend du collagène ayant subi une modification oxydative et au moins un autre des au moins deux compartiments dermiques comprend du collagène n'ayant pas subi de modification oxydative ou ayant subi une autre modification oxydative, différente du premier compartiment.

3. Equivalent de derme selon la revendication 1 ou 2, dans lequel au moins un des au moins deux compartiments dermiques comprend des fibroblastes papillaires et au moins un autre des au moins deux compartiments dermiques comprend des fibroblastes réticulaires.

4. Equivalent de peau présentant une hétérogénéité de surface comprenant un équivalent de derme selon l'une quelconque des revendications 1 à 3.

5. Procédé de préparation d'un équivalent de derme selon l'une quelconque des revendications 1 à 3, comprenant une étape consistant à préparer au moins deux lattices distinctes de compositions différentes juxtaposées longitudinalement, l'une des au moins deux lattices étant préparée à l'intérieur d'un moule et l'autre étant préparée à la périphérie du moule.

6. Procédé de préparation selon la revendication 5, dans lequel l'étape de préparation des au moins deux lattices comprend la préparation d'au moins deux solutions distinctes de compositions différentes, l'une étant pour l'intérieur du moule et l'autre étant pour la périphérie du moule.

7. Procédé de préparation d'un équivalent de peau selon la revendication 4, comprenant une étape de préparation d'un équivalent de derme par le procédé selon la revendication 5 ou 6.

8. Utilisation d'un équivalent de derme selon l'une quelconque des revendications 1 à 3 pour la préparation d'un équivalent de peau présentant une hétérogénéité de surface.

9. Utilisation d'un équivalent de derme selon l'une quelconque des revendications 1 à 3 ou d'un équivalent de peau selon la revendication 4 pour étudier les hétérogénéités de la peau telles que le vieillissement et/ou l'inflammation et/ou la pigmentation de la peau.

10. Procédé de criblage d'un composé présentant une activité après application topique sur la peau, en particulier la peau présentant des hétérogénéités, en particulier dans le domaine de l'anti-âge comme pour le traitement des rides et ridules, et/ou dans le domaine de l'inflammation et/ou dans le domaine de la pigmentation comme par exemple pour le traitement des taches pigmentaires, ledit procédé de criblage comprenant l'application d'un composé candidat sur l'équivalent de derme selon l'une quelconque des revendications 1 à 3 ou sur l'équivalent de peau selon la revendication 4.
